(19) Europäisches Patentamt
European Patent Office
Office européen des brevets

(11) Numéro de publication : **0 325 072 B1**

(12) # FASCICULE DE BREVET EUROPEEN

(45) Date de publication du fascicule du brevet : **09.10.91 Bulletin 91/41**

(21) Numéro de dépôt : **88403299.6**

(22) Date de dépôt : **23.12.88**

(51) Int. Cl.[5] : **C07C 29/40,** C07C 33/03, C07C 69/732, C07C 67/343, C07C 35/17, C07C 35/18, C07C 33/42, C07C 33/48, C07C 43/315

(54) **Perfectionnement à la préparation d'alcools tertiaires et secondaires par l'action d'un composé organo-manganeux sur un composé porteur de groupe carbonyle.**

(30) Priorité : **30.12.87 FR 8718353**

(43) Date de publication de la demande : **26.07.89 Bulletin 89/30**

(45) Mention de la délivrance du brevet : **09.10.91 Bulletin 91/41**

(84) Etats contractants désignés : **BE CH DE GB IT LI NL**

(56) Documents cités : **Eléments de la technique relevés; néant.**

(73) Titulaire : **SOCIETE NATIONALE ELF AQUITAINE**
**Tour Elf, 2, Place de la Coupole, La Défense 6**
**F-92400 Courbevoie (FR)**

(72) Inventeur : **Cahiez, Gérard**
**68 rue de Turbigo**
**F-75003 Paris (FR)**
Inventeur : **Chavant, Pierre-Yves**
**70 rue Dutot**
**F-75015 Paris (FR)**
Inventeur : **Tozzolino, Pierre**
**Chemin Carrerot Serres-Morlaas**
**F-64160 Morlaas (FR)**

(74) Mandataire : **Bertrand, Didier et al**
**Cabinet FEDIT-LORIOT 38, Avenue Hoche**
**F-75008 Paris (FR)**

EP 0 325 072 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un perfectionnement à la préparation d'alcools secondaires et tertiaires. Elle se rapporte plus spécialement à l'obtention de tels alcools par l'action d'un composé organo-manganeux sur un composé porteur d'un groupe carbonyle.

L'utilité des alcools secondaires et tertiaires est bien connue dans la synthèse de produits naturels ; parmi les méthodes classiques de leur préparation, celles qui sont basées sur l'emploi de réactifs organométalliques sont très intéressantes, parce qu'elles permettent d'obtenir des types variés d'alcools tertiaires, utiles notamment dans le domaine pharmaceutique, ainsi que dans celui des aromes et parfums. La voie par composés organométalliques a fait l'objet de nombreux travaux, comme, entre autres, ceux de BARBIER ("C.R. Acad. Sci. Paris, Vol. 128, p.110, 1899) qui décrivent la préparation d'alcools à partir d'halogénures, de magnésium et de composés carbonylés. Cette réaction est une modification de celle de WAGNER et SAYTZEEF ("Justus Liebigs Ann. Chem. Vol. 175, p.351, 1875), utilisant le zinc, et se trouvant à l'origine du remarquable développement de la chimie des organomagnésiens. L'utilisation du zinc a fait l'objet de travaux importants ; il s'agit notamment de la réaction d'un $\alpha$-bromoester et d'un composé carbonylé en présence de zinc. Cette réaction est communément appelée réaction de Reformatsky.

Des travaux récents décrivent ce même type de réaction à partir de divers métaux tels que l'étain, le cérium ou les couples Zn/Cu, Zn/Cd, Zn/Pb dans la réaction de Reformatsky.

Un progrès très net a été réalisé dans ce domaine par l'emploi d'organomanganeux, dont l'avantage réside dans leur sélectivité lors de l'attaque de molécules multi-fonctionnelles. Ainsi, selon G. CAHIEZ ("L'Actualité Chimique", Septembre 1984, p.27) les organomanganeux réagissent, comme de nombreux organométalliques, avec les aldéhydes et les cétones pour conduire aux alcools tertiaires, mais ils n'attaquent pas les esters. Cette sélectivité est intéressante puisqu'elle est totale, même à température ambiante.

Jusqu'à une période récente, les dérivés organo-manganeux étaient obtenus par échange métal-métal à partir des lithiens et magnésiens. Cette méthode de préparation interdit l'accès à des organo-manganeux fonctionnels tels que X-Mn$CH_2$-$CO_2$Et uniquement parce que le lithien ou magnésien de départ ne peut être obtenu. Cela met en évidence l'avantage qui consiste à préparer les composés organo-manganeux directement à partir du manganèse métallique et d'un halogénure organique. Récemment, HIYAMA et Coll. (Organometallics 1982, 1, 1249-1251) décrivent la réactivité du manganèse métal obtenu par réduction à l'aide d'hydrure $LiAlH_4$ du chlorure de manganèse (II), vis-à-vis de bromures allyliques ; le réactif obtenu, traité par un aldéhyde ou une cétone, conduit à l'alcool tertiaire correspondant. Selon une publication ultérieure (Chemistry Letters, p. 1237-38, 1983), l'utilisation de manganèse micronisé est économiquement plus intéressante. Cependant, l'obtention de bons rendements nécessite la mise en oeuvre de quantités excessives de réactifs : rapports métal/ cétone ou aldéhyde = 7/1, halogénure-cétone ou aldéhyde = 6. De plus, la réaction exige 1 éq. d'iode pour un reflux pendant une douzaine d'heures. Il s'agit donc là d'un ensemble de conditions qui rendent l'exploitation industrielle de telles synthèses très hypothétique.

La présente invention apporte une solution nouvelle qui permet de produire toutes sortes d'alcools secondaires ou tertiaires par l'action d'un halogénure organique, de manganèse métallique et d'un composé organique porteur d'un carbonyle, à des températures voisines de l'ambiante, et dans des bonnes conditions économiques. L'invention permet, en effet, de produire les alcools voulus bien plus vite et avec des rendements meilleurs qu'on ne pouvait le faire selon la technique antérieure.

Le procédé suivant l'invention, qui consiste à faire réagir un halogénure organique avec un composé organique, porteur d'au moins un groupe carbonyle au sein d'un solvant organique, en présence de manganèse métallique, et à hydrolyser ensuite le produit formé, est caractérisé en ce que la réaction avec le manganèse est initiée et activée par l'adjonction au milieu réactionnel de certains composés métalliques de groupe II à VIII de la Classification Périodique des Eléments, moins électropositifs que le manganèse.

Ainsi, accélère-t-on la réaction par l'addition au milieu dans le solvant organique, d'un sel ou d'une combinaison organique de métal tel que par exemple Zn, Cd, Sn, Hg, etc. Ce sel est à anion tel que sa solubilité soit suffisante dans le milieu et qu'il puisse réagir avec le manganèse métallique (formation du couple métal/métal). Le chlorure de zinc s'est révélé être particulièrement actif et économique.

Selon la nature des réactifs en présence, celle de la structure physique du Mn, et le sel métallique employé, la proportion de celui-ci peut varier entre des larges limites, en particulier entre 10 et 200% molaire par rapport au Mn et plus particulièrement entre 30 à 150% en équivalent molaire par rapport au métal. Dans une certaine mesure, le rendement en alcool à produire augmente avec la proportion de sel métallique activant employé.

Bien que le procédé de l'invention soit de préférence réalisé avec le sel métallique activant, dissous dans le solvant utilisé, il peut néanmoins être conduit avec des sels partiellement solubles maintenus en dispersion, par exemple par agitation continuelle du milieu avec le manganèse.

La granulométrie du manganèse employé est de 1 à 2000 microns et de préférence compris entre 10 et

500 microns. Il est même possible, pour des préparations à plus grandes échelles, d'utiliser des granulométries supérieures à 2 000 microns.

Les solvants, utilisables dans le procédé de l'invention, peuvent être choisis parmi tous ceux qui sont compatibles avec les réactifs en présence. On peut utiliser certains éthers, comme le THF ou le diméthoxyéthane ; conviennent également des esters, qui n'étaient pas employés jusqu'à présent, en particulier des acétates, propionates, etc. de méthyle, d'éthyle, et autres, de préférence n'ayant au total que 3 à 10 atomes de carbone. On peut également employer certains nitriles comme l'acétonitrile ou amides comme le DMF. Le tétrahydrofurane, déjà utilisé dans la technique antérieure, convient parfaitement.

Divers co-solvants peuvent être utilisés avec les solvants précités comme les solvants halogénés, notamment chlorure de méthylène, chlorobenzène, trichloroéthane, chloroalcanes, ou des hydrocarbures aliphatiques ou aromatiques.

L'ensemble des réactions qui forment le procédé de l'invention peut être représenté de la façon suivante:

(1) 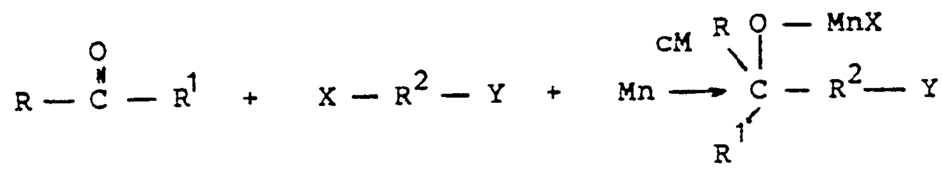

(A) (B) (C)

(2) 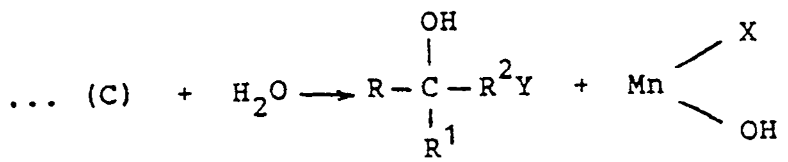

(D)

La réaction (1) a lieu au sein d'un solvant, dans lequel sont de préférence solubles les deux réactifs (A) et (B). Le principe de l'invention réside en l'addition d'un composé activant "cM", d'un métal moins électropositif que le Mn, comme expliqué plus haut.

L'organo-manganeux (C) peut être séparé du milieu réactionnel, pour être soumis à l'hydrolyse (2) ; on peut notamment évaporer le solvant après la réaction (1), et traiter le résidu à l'eau à la manière connue en soi. L'hydrolyse peut aussi avoir lieu directement, dans le solvant, après la réaction (1). Elle est en général effectuée en milieu acide, et le Mn est récupéré sous la forme de son sel $MnX_2$.

Le composé (A) est un aldéhyde lorsque $R^1$ est un H, ou une cétone quand R et $R^1$ sont des groupes hydrocarbonés ; ceux-ci peuvent être aliphatiques, de préférence en $C_1$ à $C_8$, cycloaliphatiques surtout en $C_4$ à $C_8$, ou/et aryliques, notamment phényles ou naphtyles pouvant porter des substituants alkyliques, alcényliques, halogènes ou autres.

R, dans le cas d'aldéhyde, et R ou/et $R^1$ dans celui de cétone, peuvent porter des fonctions telles que ester, nitrile, éther, sulfure, halogénure, acétal.

Ainsi, le réactif (A) peut être par exemple tel qu'acétaldéhyde, propionaldéhyde, butyraldéhyde, phénylacétaldéhyde, benzaldéhyde, aldéhyde cinnamique, anisaldéhyde, etc.

Il peut être constitué par une cétone du type acétone, dipropylcétone, méthyléthylcétone, méthylhepténone, cyclopentanone, méthylcyclohexanone, acétylpropionate de méthyle, acétophénone, benzophénone, menthone, naphtylméthylcétone, etc.

Dans l'halogénure organique (B), X désigne un halogène, surtout Br. $R^2$ est un groupe hydrocarboné ; Y représente un groupe fonctionnel, sa présence n'étant pas obligatoire. D'autre part, l'halogénure organique peut être allylique, propargylique, benzylique ou un α-halogénoester ou nitrile. Ainsi le composé (B) peut être, par exemple, $Br\text{-}CH_2\text{-}CH{=}CH_2$ ; $Cl\text{-}CH_2\text{-}CH{=}CH_2$ ; $Br\text{-}CH_2\text{-}C_6H_5$ ; $Br\text{-}CH_2\text{-}C(CH_3){=}CH_2$ ; $Br\text{-}CH_2\text{-}CO_2Et$ ; $I\text{-}CH_2\text{-}CH{=}C(CH_3)_2$ ou autres.

Les corps, cités plus haut, ne constituent que des exemples non limitatifs des composés (A) et (B), donnés seulement pour fixer les idées.

Le procédé suivant l'invention peut être réalisé avec des rapports stoechiométriques des réactifs (A), (B) et Mn dans la réaction (1). Toutefois, il s'avère préférable d'opérer avec un certain excès de métal Mn et de composé (B) par rapport au composé carbonylé (A). En effet, les rendements en l'alcool secondaire ou tertiaire sont accrus lorsque Mn et (B) sont en excès par rapport à la stoechiométrie. De façon générale, il est bon

d'employer 1 à 3 atomes Mn et 1 à 2 moles de (B) par mole de composé carbonylé (A). Les proportions préférées se situent entre 1,2 et 1,6 atomes Mn et 1,1 et 1,5 moles d'halogénure (B).

D'autre part, il est avantageux de travailler avec des solutions assez mais pas trop concentrées en composé (A), notamment des solutions 0,3 à 2 M et de préférence 0,5 à 1,6 M.

La température durant l'étape (1) du procédé peut être comprise entre 20° et 100°C, la marge de 30° à 60°C étant préférable.

L'addition des réactifs doit être effectuée pendant un temps qui est en général de l'ordre de 1 à 6 heures, et très souvent de 3 1/2 à 4 1/2 heures ; cela constitue un avantage marqué sur la technique antérieure qui exigeait environ 12 heures ou plus. Grâce à l'addition de sel métallique activateur, la réaction se déclenche immédiatement, alors qu'elle commence seulement au bout de 2 heures environ dans le procédé connu à la poudre de Mn.

L'invention est illustrée par des exemples non limitatifs, donnés plus loin, dans lesquels le mode opératoire que voici était appliqué.

Le réacteur était constitué par un tricol de 100 ml de contenance, muni d'un agitateur mécanique, monté de façon à mettre en mouvement la poudre de Mn que l'on plaçait dans le réacteur. Celui-ci comportait également un thermomètre et une tubulure d'amenée d'azote permettant d'assurer une atmosphère inerte, dans l'appareil, tout au long des opérations. Le réacteur était placé dans un bain d'eau maintenu à la température voulue.

Le manganèse était à 98-99% en poudre grossière d'une granulométrie comprise entre 10 et 500 micromètres. Il était chargé dans le tricol, avec le sel métallique activateur, et recouvert de solvant. La réaction était initiée par quelques gouttes d'halogénure organique (B), $XR^2Y$ : dès que le métal a subi un changement de coloration, et qu'un échauffement s'est produit, on commençait à introduire progressivement, à l'aide de pompes, les réactifs (A) et (B). A la fin de cette introduction, qui durait quelques heures, on continuait à agiter le milieu durant 15 à 30 mn à la même température, à laquelle a été opérée la réaction (1).

On mélangeait ensuite le milieu réactionnel avec approximativement son volume d'eau, à 20°C, légèrement acidifiée avec de l'HCl de façon à amener le pH de l'eau à 7 ou à une valeur un peu inférieure, pour dissoudre les sels métalliques présents.

Le produit recherché était extrait à l'éther et lavé au bicarbonate de Na.

Le rendement en alcool obtenu, par rapport au composé (A), était calculé à partir de la quantité d'alcool isolé par extraction et distillation.

EXEMPLES 1 et 2

Préparation de l'allyl-4 heptanol-4 comparativement selon la technique antérieure et suivant l'invention

En opérant comme indiqué plus haut, on fait réagir 1 mole de dipropyl cétone $CH_3CH_2CH_2$-CO-$CH_2CH_2$-$CH_3$ avec 1,1 mole de bromure d'allyle $CH_2$=CH-$CH_2Br$ et 1,3 atomes de Mn métallique en poudre, dans du tétrahydrofurane bien sec à la proportion de 0,7 litre de ce solvant pour les quantités susindiquées. La réaction a lieu à 60°C pendant 4 heures.

EXEMPLE 1

Rien d'autre n'est ajouté au milieu réactionnel. A 60°C, la réaction ne se déclenche pas après 6 heures. L'analyse du milieu réactionnel ne permet pas de mettre en évidence la formation du produit attendu.

EXEMPLE 2

Au mélange réactionnel, décrit plus haut, on a ajouté, au départ, 2,7 g de $ZnCl_2$ par litre de solution, soit 0,02 mole. La réaction se déclenche dès qu'on commence à introduire les réactifs. Le rendement après 4 heures à 60°C, en l'alcool tertiaire est de 77% par rapport à la dipropylcétone. L'activation par le chlorure de zinc est donc remarquable.

EXEMPLE 3

Influence de la proportion de sel métallique activant

On a préparé de l'allyl-6 undécanol-6

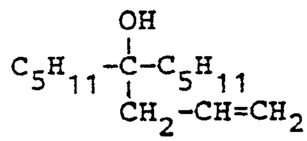

comme dans l'exemple 2, mais en remplaçant la dipropylcétone par la dipentyl-cétone $(C_5H_{11})_2CO$, et le bromure par le chlorure d'allyle.

Avec différentes proportions de $ZnCl_2$ ajouté, en mol. $ZnCl_2$ par mole de cétone, on a trouvé les rendements suivants sur la dipentyl cétone, déterminés à partir de l'alcool tertiaire formé, isolé.

| Moles $ZnCl_2$ par mole de cétone | Rendement % |
|---|---|
| 0,02 | 85 |
| 0,05 | 89 |
| 0,10 | 90 |
| 0,15 | 95 |

EXEMPLE 4

Rôle de la température

L'exemple 2 est répété à trois températures différentes pour le premier stade de la préparation, la concentration de la dipropyl cétone étant de 1,33 M dans le tétrahydrofurane.

Les rendements sur la cétone, déterminés par chromatographie, sont :

| | |
|---|---|
| pour 50°C | 60% |
| 60° | 77 |
| 70° | 85 |

EXEMPLE 5

Influence des proportions de réactifs

Des préparations identiques à celles de l'exemple 4 sont effectués avec des proportions variables de bromure (B) et de Mn. Elles ont conduit aux rendements suivants indiqués par la chromatographie en phase vapeur.

| Moles $CH_2=CH-CH_2Br$ par mole de cétone | Atomes Mn par mol. cétone | Rendement % |
|---|---|---|
| 1,1 | 1,3 | 77 |
| 1,3 | 1,4 | 84 |
| 1,4 | 1,5 | 90 |

EXEMPLE 6

Effet de la concentration en composé (A)

En répétant l'exemple 2 avec les concentrations molaires variables de dipropyl cétone (composé A), on a trouvé les rendements suivants.

| Concentration de (A) dans le milieu réactionnel | | Rendement % |
|---|---|---|
| 0,67 M | .............. | 76 |
| 1,33 | .............. | 79 |
| 1,70 | .............. | 75 |

Il existe donc un certain optimum.

## EXEMPLE 7

En remplaçant, dans l'exemple 5, le bromure d'allyle par le chlorure (1,1 moles), et en ajoutant 0,1 mol $ZnCl_2$, au lieu de 0,02 mol. par mole de dipropyl cétone, on a pu atteindre les rendements de

81% avec 1,3 atomegr Mn/mole cétone
et 94% " 1,8 " " "

## EXEMPLE 8

### Application à une cétone-ester

Dans les conditions générales de l'exemple 2, on a fait réagir 1 mole de oxo-8 dodécanoate de méthyle avec du chlorure d'allyle en présence de Mn et de 0,1 mole de $ZnCl_2$, à 60°C, pendant 4 h 1/4. On a ainsi obtenu, suivant la réaction

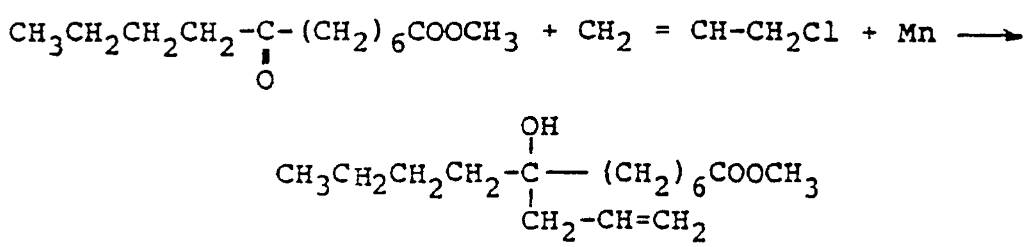

Le rendement en cet alcool tertiaire à groupe insaturé et groupe ester est de 67% sur la cétone de départ, lorsqu'on utilise 1,3 mol de chlorure d'allyle avec 1,5 atome Mn. On peut arriver à 76% en employant 1,6 mole de chlorure et 1,6 atome Mn.

Cette préparation donne de très mauvais résultats si le milieu n'est pas additionné d'un sel de métal, notamment $ZnCl_2$ ; ainsi l'invention permet-elle l'obtention aisée d'alcools porteurs de deux autres fonctions.

## EXEMPLE 9

### Application à des aldéhydes

Suivant le mode opératoire de l'exemple 2, à 60°C, on a fait réagir 1 mole d'aldéhyde hexylique (heptanal normal) avec 1,3 mole de chlorure d'allyle et 1,3 atome de Mn, la concentration de l'aldéhyde dans le tétrahydrofurane étant 0,67 M. L'activateur $ZnCl_2$ était présent à raison de 0,1 mole par mole d'aldéhyde.

Aprés 4 h 10 mn, le rendement en l'alcool secondaire

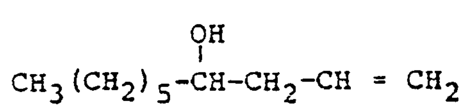

était de 85% par rapport à l'aldéhyde de départ.

## EXEMPLE 10

Application à un halogénure à fonction ester

Le mode opératoire précédent fut appliqué à la réaction

$$(CH_3CH_2CH_2CH_2)_2CO + BrCH_2COOC_2H_5 + Mn \rightarrow$$

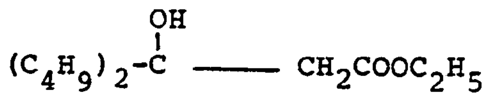

et a conduit à un rendement de 23%.

## EXEMPLES 11 à 24

Dans le tableau annexé sont donnés les rendements en alcool secondaire ou tertiaire de quelques préparations faites à partir de divers composés carbonylés (A) et halogénures organiques (B). Les formules de ces composés, ainsi que les proportions de (B) et de Mn par mole de (A) y sont indiquées. La température était de 60°C, la quantité de chlorure de zinc de 0,1 mole par mole de (A) et la durée de 4h à 4h 1/2. Le solvant est le THF.

TABLEAU RECAPITULATIF DES EXEMPLES 11 à 24

| Ex. n° | Composé (A) | Halogénure (B) | Mn | Rendement % | Alcool obtenu |
|---|---|---|---|---|---|
| 11 | 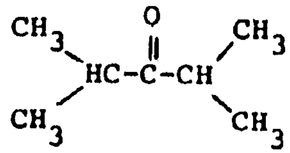 | $ClCH_2-CH=CH_2$ 1,3 | 1,3 | 78 | 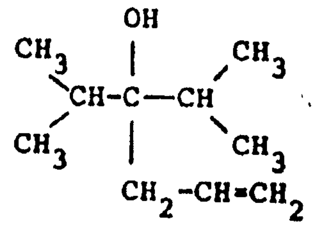 |
| 12 | 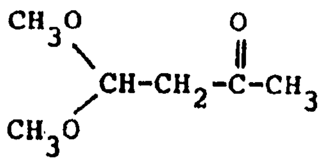 | " | " | 77 | 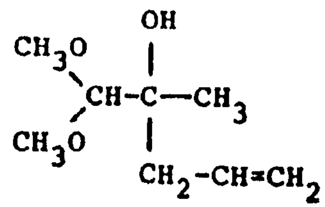 |
| 13 | 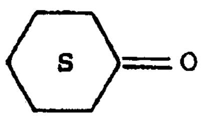 | " | 1,5 | 81,5 | 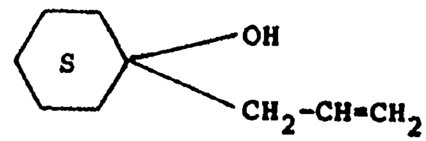 |
| 14 | 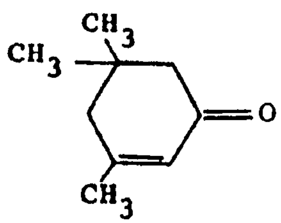 | " | 1,3 | 94 | 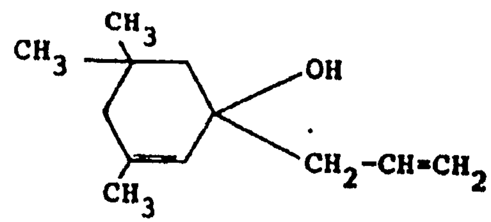 |

TABLEAU RECAPITULATIF (suite)

| Ex. n° | Composé (A) | Halogénure (B) | Mn | Rendement % | Alcool obtenu |
|---|---|---|---|---|---|
| 15 | 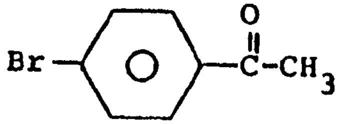 | $ClCH_2-CH=CH_2$ 1,3 | 1,5 | 76 | 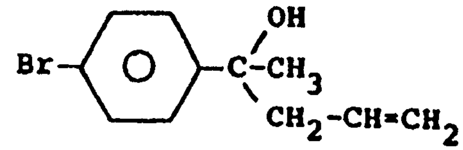 |
| 16 | 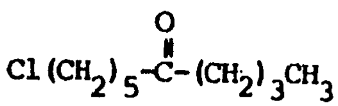 | " | 1,3 | 93 | 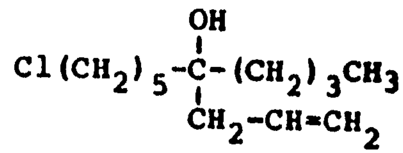 |
| 17 | 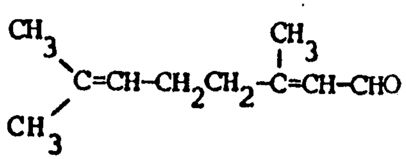 | $CH_2=CH-CH_2Cl$ 1,3 | 1,3 | 80 | 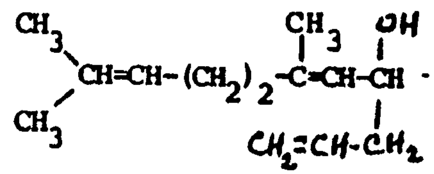 |
| 18 | 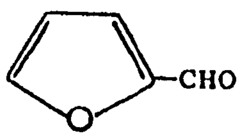 | " | " | 94 | 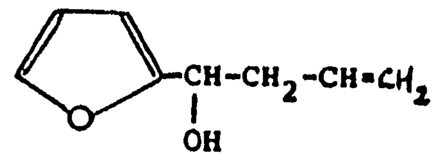 |
| 19 | 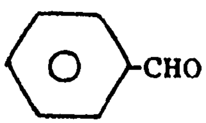 | " | 1,7 | 90 | 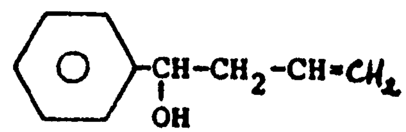 |

TABLEAU RECAPITULATIF (suite)

| Ex.n° | Composé (A) | Halogénure (B) | Mn | Rendement % | Alcool obtenu |
|---|---|---|---|---|---|
| 20 | 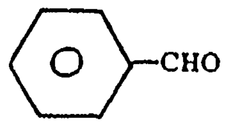 | 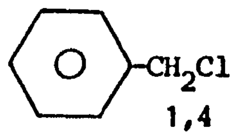 1,4 | 1,5 | 88 | 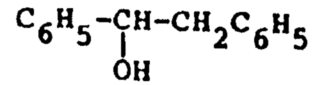 |
| 21 | $CH_3(CH_2)_3CHO$ | 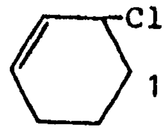 1,3 | 1,3 | 85 | 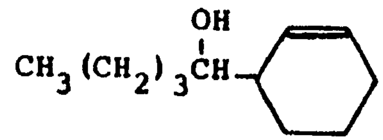 |
| 22 | $CH_3(CH_2)_5CHO$ | 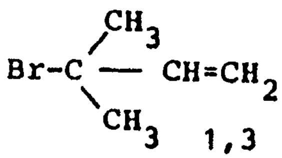 1,3 | 1,5 | 76 | 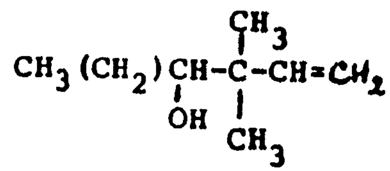 |
| 23 | $(CH_3O)_2CHCH_2COCH_3$ | 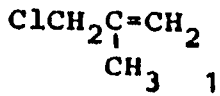 1,3 | 1,3 | 95 | 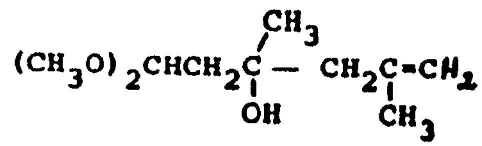 |
| 24 | $Cl(CH_2)_5CO(CH_2)_3CH_3$ | $ClCH_2CH=CH_2$ 1,3 | 1,3 | 93 | 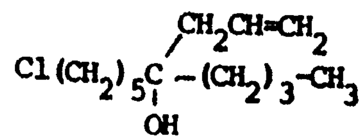 |

EXEMPLES 25 à 32

Des préparations, similaires à celles de l'exemple 2, à partir de la dipropylcétone $(C_3H_7)_2CO$, ont été effectuées avec différents halogénures organiques et dans divers solvants. En outre, le sel de zinc a été remplacé par des sels de cadmium, de mercure ou d'étain.

Voici les caractéristiques de ces essais.

| Essai n° | Halogénure | Composé métallique | | Solvant |
|---|---|---|---|---|
| 25 | 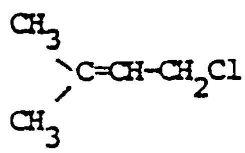 | $HgCl_2$ | 0,1 mole | THF |
| 26 | " | $HgCl_2$ | 0,05 mole | Acétate d'éthyle |
| 27 | " | $CdCl_2$ | 0,1 mole | THF |
| 28 | " | $SnCl_4$ | 0,1 mole | THF |
| 29 | $C_6H_5CH_2Cl$ | $HgCl_2$ | 0,1 mole | THF |
| 30 | " | $CdCl_2$ | 0,1 mole | THF |
| 31 | $CH_2{=}C(CH_3)CH_2Br$ | $HgCl_2$ | 0,1 mole | THF |
| 32 | " | $ZnCl_2$ | 0,05 mole | Acétate d'éthyle |

Les rendements sont du même ordre que ceux de l'exemple 2.

## Revendications

1. Procédé de préparation d'alcools secondaires ou tertiaires par l'action d'un halogénure organique sur du manganèse métallique, en présence d'un composé carbonylé au sein d'un solvant, suivie d'une hydrolyse, caractérisé en ce qu'un composé d'un métal de groupe II à VIII de la Classification Périodique des Eléments, moins électropositif que le manganèse, est présent dans le milieu réactionnel.

2. Procédé suivant la revendication 1, caractérisé en ce que le composé d'un métal est celui de Zn, Cd, Hg ou Sn.

3. Procédé suivant la revendication 1 ou 2, caractérisé en ce que le composé d'un métal est un sel à anion organique ou inorganique, en particulier chlorure, bromure ou iodure, notamment ceux de zinc.

4. Procédé suivant la revendication 1 ou 2, caractérisé en ce que le composé d'un métal est une combinaison organométallique.

5. Procédé suivant une des revendications précédentes, caractérisé en ce que la quantité de composé d'un métal ajouté est d'environ 0,01 à 2 équivalents par rapport au métal et plus particulièrement 0,3 à 1,5 équivalents molaires.

6. Procédé suivant une des revendications précédentes, caractérisé en ce que l'on fait réagir 1 mole de cétone ou d'aldéhyde avec 1 à 2 moles d'halogénure organique et 1 à 3 atome-grammes de manganèse, la concentration préférée en cétone ou en aldéhyde, dans le milieu réactionnel, étant de 0,3 à 2 M et particulièrement 0,5 à 1,6 M.

7. Procédé suivant une des revendications précédentes, caractérisé en ce que le solvant est un éther, un ester, un nitrile, un amide ou une combinaison de ceux-ci avec un solvant chloré ou un hydrocarbure aliphatique ou aromatique.

8. Procédé suivant une des revendications précédentes, caractérisé en ce que la réaction entre le composé carbonylé, l'halogénure organique et le manganèse a lieu entre 0° et 100°C et de préférence entre 50 et 80°C.

9. Procédé suivant une des revendications précédentes, caractérisé en ce que le composé carbonylé est du type R-CO-$R^1$ où R et $R^1$ sont des groupes hydrocarbonés aliphatiques, de préférence en $C_1$ à $C_8$, cycloaliphatiques surtout en $C_4$ à $C_8$, ou aryliques, notamment phényles ou naphtyles, pouvant porter des substituants alkyliques, alcéniques, alcyniques ou divers groupes fonctionnels tels que ester, amide ou nitrile, entre autres, R pouvant également être un hydrogène.

10. Procédé suivant une des revendications précédentes, caractérisé en ce que l'halogénure organique est un composé X-$R^2$-Y activé, X étant un halogène, $R^2$ représentant un groupe hydrocarboné, notamment alkyle, alcényle, alcynyle ou aryle, et Y un groupe fonctionnel tel que ester, nitrile ou amide.

11. Procédé suivant une des revendications précédentes, caractérisé en ce que le solvant est de l'acétate d'éthyle.

## Patentansprüche

1. Verfahren zur Herstellung von sekundären oder tertiären Alkoholen durch Einwirkung eines Organohalogens auf metallisches Mangan in Gegenwart einer Carbonylverbindung in einem Lösungsmittel, gefolgt von der Hydrolyse, dadurch gekennzeichnet, daß eine Metallverbindung der Gruppe II-VIII des Periodensystems der Elemente, das weniger elektropositiv als das Mangan ist, im Reaktionsmedium vorhanden ist.

2. Verfahren nach Anspruch 1, dadurch gekennzeichnet, daß die Metallverbindung eine von Zn, Cd, Hg oder Sn ist.

3. Verfahren nach Anspruch 1 oder 2, dadurch gekennzeichnet, daß die Metallverbindung ein Salz eines organischen oder anorganischen Anions ist, besonders von Chlor, Brom oder Jod, insbesondere die von Zink.

4. Verfahren nach Anspruch 1 oder 2, dadurch gekennzeichnet, daß die Metallverbindung eine organometallische Kombination ist.

5. Verfahren nach einem der vorstehenden Ansprüche, dadurch gekennzeichnet, daß die Menge an zugefügter Metallverbindung ungefähr 0,01 bis 2 Äquivalente in bezug auf das Metall, und besonders bevorzugt 0,3 bis 1,5 Moläquivalente beträgt.

6. Verfahren nach einem der vorstehenden Ansprüche, dadurch gekennzeichnet, daß man 1 Mol Keton oder Aldehyd mit 1 bis 2 Molen organischem Halogenid und 1 bis 3 Grammatomen Mangan umsetzt, wobei die bevorzugte Konzentration an Keton oder an Aldehyd im Reaktionsmilieu 0,3 bis 2 M und insbesondere 0,5 bis 1,6 M beträgt.

7. Verfahren nach einem der vorstehenden Ansprüche, dadurch gekennzeichnet, daß das Lösungsmittel ein Ether, ein Ester, ein Nitril, ein Amid oder eine Kombination davon mit einem chlorierten Lösungsmittel oder einem aliphatischen oder aromatischen Kohlenwasserstoff ist.

8. Verfahren nach einem der vorstehenden Ansprüche, dadurch gekennzeichnet, daß die Reaktion zwischen der Carbonylverbindung, dem organischen Halogenid und dem Mangan zwischen 0° und 100°C und vorzugsweise zwischen 50 und 80°C stattfindet.

9. Verfahren nach einem der vorstehenden Ansprüche, dadurch gekennzeichnet, daß die Carbonylverbindung vom Typ R-CO-$R^1$ ist, worin R und $R^1$ aliphatische, vorzugsweise von $C_1$ bis $C_8$, cycloaliphatische, vor allem von $C_4$ bis $C_8$, oder Arylkohlenwasserstoffe, insbesondere Phenyle oder Naphthyle, sind, die Alkyl-, Alkenyl-, Alkinylsubstituenten oder verschiedene funktionelle Gruppen, wie unter anderem eine Ester-, Amid- oder Nitrilgruppe tragen können, wobei R gleichermassen ein Wasserstoff sein kann.

10. Verfahren nach einem der vorstehenden Ansprüche, dadurch gekennzeichnet, daß das organische Halogenid eine aktivierte Verbindung X-$R^2$-Y ist, worin X ein Halogen ist, $R^2$ eine Kohlenwasserstoffgruppe darstellt, insbesondere Alkyl, Alkenyl, Alkinyl oder Aryl, und Y eine funktionelle Gruppe ist, wie eine Ester-, Nitril- oder Amidgruppe.

11. Verfahren nach einem der vorstehenden Ansprüche, dadurch gekennzeichnet, daß das Lösungsmittel Ethylacetat ist.

## Claims

1. Method of preparing secondary or tertiary alcohols by the action of an organic halide on metallic manganese, in the presence of a carbonyl compound in a solvent, followed by hydrolysis, characterised in that a compound of a metal in groups II to VIII of the Periodic Table, less electropositive than manganese, is present in the reaction medium.

2. Method according to claim 1, characterised in that the metal compound is a compound of Zn, Cd, Hg, or Sn.

3. Method according to claim 1 or 2, characterised in that the metal compound is a salt with an organic or inorganic anion, in particular a chloride, bromide or iodide, notably of zinc.

4. Method according to claim 1 or 2, characterised in that the metal compound is an organo-metallic combination.

5. Method according to one of the preceding claims, characterised in that the quantity of metal compound added is approximately 0.01 to 2 equivalents compared with the metal and more especially 0.3 to 1.5 molar equivalents.

6. Method according to one of the preceding claims, characterised in that one mole of ketone or aldehyde is reacted with 1 to 2 moles of organic halide and 1 to 3 gram atoms of manganese, the preferred concentration of ketone or aldehyde in the reaction medium being 0.3 to 2 M and especially 0.5 to 1.6 M.

7. Method according to one of the preceding claims, characterised in that the solvent is an ether, ester, nitrile, amide or a combination of these with a chlorinated solvent or an aliphatic or aromatic hydrocarbon.

8. Method according to one of the preceding claims, characterised in that the reaction between the carbonyl compound, the organic halide and the manganese takes place between 0° and 100°C and preferably between 50 and 80°C.

9. Method according to one of the preceding claims, characterised in that the carbonyl compound is of the type $R-CO-R^1$, where R and $R^1$ are aliphatic hydrocarbon groups, preferably $C_1$ to $C_8$, cycloaliphatic, especially $C_4$ to $C_8$, or aryl, notably phenyls or naphtyls, able to carry alkyl, alkenyl or alkynyl substituents or various functional groups such as ester, amide or nitrile, amongst others, R also being able to be a hydrogen atom.

10. Method according to one of the preceding claims, characterised in that the organic halide is an activated compound $X-R^2-Y$, X being a halogen, $R^2$ representing a hydrocarbon group, notably alkyl, alkenyl, alkynyl or aryl, and Y a functional group such as ester, nitrile or amide.

11. Method according to one of the preceding claims, characterised in that the solvent is ethyl acetate.